# EUROPÄISCHE PATENTSCHRIFT

(11) **EP 2 833 951 B1**
(45) Veröffentlichungstag und Bekanntmachung des Hinweises auf die Patenterteilung: **24.10.2018**
(21) Anmeldenummer: 13715940.6
(22) Anmeldetag: 02.04.2013
(51) Int. Cl.: A61M 15/00

(54) **VORRICHTUNG ZUR PORTIONIERTEN AUSGABE VON MEDIKAMENTEN**
DEVICE FOR THE PORTIONED OUTPUT OF MEDICATIONS
DISPOSITIF DE DISTRIBUTION DE MÉDICAMENTS PAR PORTIONS

(30) Priorität: 05.04.2012 DE 102012102974; 05.06.2012 DE 102012104850
(43) Veröffentlichungstag der Anmeldung: 11.02.2015
(73) Patentinhaber: Von Schuckmann, Alfred, 47627 Kevelaer (DE)
(72) Erfinder: Von Schuckmann, Alfred, 47627 Kevelaer (DE)
(74) Vertreter: Müller, Enno
(86) Internationale Anmeldenummer: PCT/EP2013/056927
(87) Internationale Veröffentlichungsnummer: WO 2013/150021

(56) Entgegenhaltungen:
- DE-A1- 4 106 379
- FR-A1- 2 961 186
- US-A- 5 582 162

## Beschreibung

Die Erfindung betrifft eine Vorrichtung zur portionierten Ausgabe von Medikamenten gemäß Gattungsbegriff des Hauptanspruches.

Vorrichtungen der in Rede stehenden Art sind beispielsweise bekannt durch die DE-OS 4106379. Diese sind ausgelegt zur handbedienbaren Ausgabe von portionierten Medikamenten, insbesondere von inhalierfähigen, pulverförmigen Medikamenten. Hierzu ist in der Vorrichtung ein transportierbarer Bandstreifen vorgesehen, im Wesentlichen bestehend aus einem Basisstreifen, in welchem vereinzelte, in Streifen-Transportrichtung zueinander gleichmäßig beabstandete Kammern zur Aufnahme von Medikamentenportionen ausgeformt sind, und einem Verschlussstreifen, der die mit dem Medikament befüllten Kammern versiegelt und geeignet ist, vom Basisstreifen abgezogen zu werden. Durch Freilegen einer Kammer infolge Abziehen des Verschlussstreifens wird das in der Kammer bevorratete, portionierte Medikament zur Ausgabe freigegeben. Die in dem Basisstreifen ausgebildeten Kammern sind hierbei erhaben gegenüber der Streifenbasis, die bevorzugt flächig ausgebildet insbesondere auf der der Kammererhebung abgewandten Seite die Kontaktfläche zu dem Verschlussstreifen anbietet.

Zum Stand der Technik ist im Weiteren auch auf die US 5,582,162 A und die FR 2 961186 A1 verweisen.

Im Hinblick auf den vorbeschriebenen Stand der Technik stellt sich der Erfindung die Aufgabe, eine Vorrichtung der in Rede stehenden Art anzugeben, die bei einfacher Bauform störungsfrei betätigbar ist und auch mit weniger stabilen Bandstreifen auskommt.

Diese Aufgabe ist beim Gegenstand des Anspruches 1 gelöst, wobei darauf abgestellt ist, dass die Betätigung der Handhabe über ein Sperrglied durch eine in Richtung auf eine Mundstücköffnung gerichtete Kammererhebung blockierbar ist, in welcher Stellung eine der blockierenden Kammererhebung bewegungsmäßig vorgeordnete, vom Verschlussstreifen befreite Kammer oberhalb einer Durchfallöffnung liegt, welche Öffnung mittels einer von einem Saugluftstrom in die Öffnungsstellung bewegbaren Klappe verschließbar ist.

Die Kammer ist zufolge Abziehen des Verschlussstreifens vom Basisstreifen und Umlenkung des Verschlussstreifens zu einer nachfolgenden, bevorzugten Saugluftentleerung durch den Benutzer nahezu durch normales Einatmen, freigelegt. Die Kammer wird an der Unterseite geöffnet. Der Kammerinhalt erfährt eine Schwerkraft-Erdbeschleunigung zusätzlich zum Luftsaug-Effekt. Das Gerät ist bestens geeignet für die Überkopfhaltung.

Eine positionsgenaue Lage der zu entleerenden Kammer ist bevorzugt durch das Sperrglied für die Drehhandhabe gegeben, in welcher Sperrstellung die der steuernden Kammer bewegungsmäßig vorgeordnete Kammer durch Umlenkung des Verschlussstreifens zur Saugluftentleerung über der Durchfallkammer freiliegt. Das Verlagerungsmaß des Bandstreifens zum Anbieten einer nächsten, gefüllten Kammer wird bevorzugt unmittelbar abgegriffen an den Kammern, indem das vorgesehene Sperrglied über die sich über die Streifenbasis des Bandstreifens beziehungsweise des Basisstreifens erhebende Kammer bewegt wird. Es wird entsprechend unmittelbar das Abstandsmaß zweier in Bewegungsrichtung des Bandstreifens hintereinander liegender Kammern als Maß zur Verlagerung des Bandstreifens zum Anbieten der nächsten, gefüllten Kammer genutzt. Dies bietet weiter den Vorteil, dass in der Vorrichtung gegebenenfalls auch verschiedene Basisstreifen mit unterschiedlichen Abständen oder ein Basisstreifen mit unterschiedlichen Kammerabständen von in Bewegungsrichtung nachfolgenden Kammern genutzt werden können.

Um einem schwerkraftbedingten Herausfallen der Medikamentenportion in den saugluftdurchströmbaren Ausgabebereich der Vorrichtung vor Durchführung der Inhalation entgegenzuwirken, ist diese hinsichtlich des Verschlussstreifens freigelegte Kammer zunächst durch die Klappe verschlossen. Erst mit Einsetzen der Saugluftströmung zufolge eines durchgeführten Inhalationsvorganges durch den Benutzer wird die Klappe in eine die vorrichtungsseitige Durchfallöffnung und somit die Kammeröffnung zugleich freigebende Stellung bewegt, bevorzugt schwenkverlagert, wonach die Medikamentenportion bevorzugt schwerkraftbegünstigt aus der Kammer in den Ausgabe- und Strömungsbereich ausrieselt und durch die vorherrschende Saugluftströmung zur Inhalation transportiert wird.

Das Ausräumen der Kammer wird hierbei weiter bevorzugt unterstützt durch eine durch die Kammer geführte Saugluftverwirbelung. Die Klappe ist weiter bevorzugt in eine die Durchfallöffnung verschließende Stellung vorgespannt, insbesondere federvorgespannt. Diese Vorspannung resultiert in bevorzugter Ausgestaltung allein oder zumindest zum größten Teil durch das Material der Klappe selbst, welche in weiter bevorzugter Ausgestaltung aus einem gummielastischen Material hergestellt ist.

Insbesondere ist hierbei eine im Wesentlichen schmiegsame Klappe vorgesehen, deren aus der Elastizität resultierende Rückstellkraft bevorzugt allein durch die im Zuge der Inhalation vorherrschende Saugluftströmung überwunden werden kann. Alternativ ist beispielsweise eine in sich starre Klappe vorgesehen, die weiter beispielsweise über eine gesonderte Feder, wie z.B. eine Schenkelfeder oder Druckfeder, belastet ist. Zufolge Anordnung einer solchen Klappe ist sichergestellt, dass eine Ausgabe des Medikaments in die saugluftdurchströmbare Ausgabekammer erst zum Zeitpunkt der tatsächlichen Inhalation erfolgt. Darüber hinaus ist hierdurch in vorteilhafter Weise einer beispielsweise Doppelportionierung entgegengewirkt.

In einer weiter bevorzugten Ausgestaltung ist eine Anfangs-Stopp-Stellung des Verschlussstreifens vorgesehen, kurz bevor die vorderste Kammer die Umlenkkante erreicht und bevor die nachfolgende Kammer das Sperrglied betätigt. Diese Anfangs-Stopp-Stellung ist bevorzugt die Auslieferungsstellung der Vorrichtung. Die vorderste Kammer liegt hierbei in einer insbesondere zur Durchfallöffnung beabstandeten Position, weiter insbesondere in einer zur Umlenkkante beabstandeten Position. Die Kammer ist durch den Verschlussstreifen in dieser Anfangsposition weiterhin dichtend verschlossen. Der in Bewegungsrichtung des Bandstreifens betrachtete Abstand zwischen zwei Kammern, insbesondere zwischen der in Bewegungsrichtung ersten und zweiten Kammer, ist auf die Erlangung insbesondere dieser Anfangs-Stopp-Stellung angepasst.

Zudem ist bevorzugt, dass der Bandstreifen mit den verschlossenen Kammern in einer Wickelkammer aufgewickelt ist und der sich jenseits der Umlenkstelle fortsetzende Basisstreifen sich in eine vergrößerte Einschubkammer einschiebt, während der Verschlussstreifen sich auf einem von der Drehhandhabe angetriebenen Dorn aufwickelt. Bevorzugt ist entsprechend lediglich eine aktiv über die Drehhandhabe drehbare Aufwickelvorrichtung vorgesehen, nämlich weiter bevorzugt der Dorn zum Aufwickeln des von dem Basisstreifen abgezogenen Verschlussstreifens.

Eine insbesondere konstruktiv günstige, weiter insbesondere hinsichtlich der Gesamtabmessungen der Vorrichtung sich als günstig erweisende Ausbildung sieht vor, dass der Dorn sich zwischen der Wickelkammer und der Einschubkammer erstreckt. Es ist hierbei bevorzugt grundrissmäßig - bei einer sich bevorzugt senkrecht zur Drehachse des Dornes erstreckenden Grundrissfläche - quasi eine Ineinanderschachtelung von Wickelkammer, Einschubkammer und einer, den Dorn aufweisenden Verschlussstreifen-Wickelkammer vorgesehen.

In diesem Zusammenhang ist weiter bevorzugt, dass das Sperrglied, die Durchfallöffnung und der Dorn im Wesentlichen auf einer Radialen der Drehhandhabe beziehungsweise der Drehachse des Dornes hintereinander liegen, weiter insbesondere in einem von der Dornachse radial sich erstreckenden Winkelbereich von 0 bis 30°, weiter bevorzugt 0 bis 15°.

Die Vorrichtung weist zur gezielten Weiterverlagerung des Bandstreifens ein Schrittschaltwerk auf, wobei das Sperrglied bevorzugt Teil dieses Schrittschaltwerkes ist. In der Zusammenwirkungsstellung, in welcher das Sperrglied gegen die Erhebung einer in Bewegungsrichtung der ausgebenden Kammer nachgeschalteten Kammer tritt, ist das Sperrglied in eine insbesondere die Drehhandhabe sperrende Stellung verlagert. Die Sperrung belastet jedoch nicht störend die Wand der Kammererhebung, weil die Lagerstelle des Sperrgliedes eine etwa noch ausgeübte Belastung der Drehhandhabe aufnimmt. Auch kann der abtastende Arm des Sperrgliedes federnd beweglich sein. Bevorzugt ist diesbezüglich ein zweiarmiges Sperrglied, wobei ein Arm zur Zusammenwirkung mit der Kammererhebung und der weitere Arm zur Zusammenwirkung mit einer Verzahnung der Drehhandhabe ausgebildet ist.

Die Drehhandhabe ist bevorzugt als großflächige Scheibe ausgebildet, die handhabungsgünstig zumindest eine Teilfläche der Gehäuseseitenwand überdeckend angeordnet ist.

Um insbesondere nach erfolgter Inhalation zur Vorbereitung einer nächsten Inhalation diese Sperrstellung der Drehhandhabe aufzuheben, ist in einer bevorzugten Ausgestaltung vorgesehen, dass das abtastende Sperrglied von einem Vorsprung einer Mündungs-Verschlusskappe in die Vorbereitungsstellung zum Abtasten der nächsten Kammer zurückstellbar ist. Entsprechend ist die Verschlusskappe der Vorrichtung zum Verschließen des im Zuge der Inhalation lippenumschlossenen Mündungsstutzens zugleich Funktionsteil zur Nutzung der Vorrichtung. Die Verschlusskappe dient zur Vorbereitung der Vorrichtung für eine nächste Inhalation, indem bevorzugt ein Vorsprung der Verschlusskappe entweder den Mündungsstutzen durchsetzend oder auch außerhalb des Mündungsstutzens verlaufend und eine entsprechende Gehäusewandung durchsetzend auf das Sperrglied derart einwirkt, dass der Abtastabschnitt des Sperrgliedes in eine zur Abtastung der nachfolgenden Kammer vorbereitende Stellung verlagert wird, dies einhergehend mit einer Aufhebung der Sperrung der Drehhandhabe durch das Sperrglied.

Da die Mündungs-Verschlusskappe in der bevorzugten Ausführungsform zugleich Funktionsteil der Vorrichtung, insbesondere des Schrittschaltwerkes ist, ist dieses weiter bevorzugt unverlierbar an der Vorrichtung gehaltert. Bevorzugt ist zudem, dass die Mündungs-Verschlusskappe längsverschieblich und schwenkbar auf den Mündungsstutzen aufsetzbar ist. Die Längsverschiebbarkeit ermöglicht das aufschiebende, im Wesentlichen lineare Aufsetzen der Kappe auf den Mündungsstutzen, diesen bevorzugt kappenartig umgebend und überdeckend, wobei im Zuge dieser linearen Aufsetzbewegung bevorzugt der Vorsprung der Verschlusskappe auf das abtastende Sperrglied zur Verlagerung desselben in die Entsperrposition einwirkt. Die Verschwenkbarkeit der Verschlusskappe ermöglicht dem Benutzer, diese in eine den Inhalationsvorgang, insbesondere das Umschließen des Mündungsstutzens mit den Lippen nicht störende Position zu verlagern.

In einer weiter bevorzugten Ausgestaltung ist vorgesehen, dass ein Vorsprung der Mündungs-Verschlusskappe die Drehhandhabe sperrt. Entsprechend ist in der Mündungstützen-Verschlussstellung, d.h. weiter bevorzugt in der Nichtnutzungsstellung der Vorrichtung, ein Drehen der Handhabe und damit einhergehend eine Weiterverlagerung des Bandstreifens unterbunden, dies obwohl das abtastende Sperrglied durch den weiteren Vorsprung der Verschlusskappe in eine nicht sperrende Stellung verlagert ist. Bevorzugt wirkt hierbei der Sperrvorsprung der Verschlusskappe auf dieselbe Zahnreihenausformung der Drehhandhabe oder eines durch die Drehhandhabe drehend mitgeschleppten Vorrichtungselements ein, auf das auch in der Nutzungsstellung der Vorrichtung das Sperrglied zum Eingriff ausgelegt ist. Die Sperrung der Drehhandhabe ermöglicht auch, diese recht groß und so auch für Kinderhand bedienbar zu machen.

Bevorzugt ist zudem ein in Richtung auf die Einschubkammer weisender Einführabschnitt vorgesehen. Dieser ist bevorzugt in Bewegungsrichtung des Basisstreifens der Einschubkammer vorgelagert, weiter bevorzugt den Eintrittsbereich der Einschubkammer bildend.

In bevorzugter Ausgestaltung ist der Einführabschnitt schanzenartig ausgebildet, zur weiter bevorzugten Lenkung des Basisstreifens in einen vom Einführabschnitt abgewandten Bereich der Einschubkammer. Hierdurch ist in vorteilhafter Weise ein stapelartiges Ablegen des Basisstreifens in der Einschubkammer unterstützt.

Die vor- und nachstehend angegebenen Bereiche bzw. Wertebereiche oder Mehrfachbereiche schließen hinsichtlich der Offenbarung auch sämtliche Zwischenwerte ein, insbesondere in 1/10- Schritten der jeweiligen Dimension, ggf. also auch dimensionslos, insbesondere 1,01-Fach etc. einerseits zur Eingrenzung der genannten Bereichsgrenzen von unten und/oder oben, alternativ oder ergänzend aber auch im Hinblick auf die Offenbarung eines oder mehrerer singulärer Werte aus dem jeweils angegebenen Bereich.

Der mehrfach umgelenkte Saugluftstrom begünstigt die Durchmischung.

Nachstehend ist die Erfindung anhand der beigefügten Zeichnung erläutert, die aber lediglich ein Ausführungsbeispiel (Ausführungsbeispiele) darstellt. Ein Teil, das nur bezogen auf eines der Ausführungsbeispiele erläutert ist und bei einem weiteren Ausführungsbeispiel aufgrund der dort herausgestellten Besonderheit nicht (gerade) durch ein anderes Teil ersetzt ist, ist damit auch für dieses weitere Ausführungsbeispiel als jedenfalls mögliches vorhandenes Teil beschrieben. Auf der Zeichnung zeigt:
- Fig. 1: eine Vorrichtung der in Rede stehenden Art in perspektivischer Darstellung mit Blick auf eine, eine Drehhandhabe aufweisende Vorderseite, eine Nichtbenutzungsstellung der Vorrichtung betreffend;
- Fig. 2: eine der Fig. 1 entsprechende Darstellung, jedoch mit Blick auf die Rückseite;

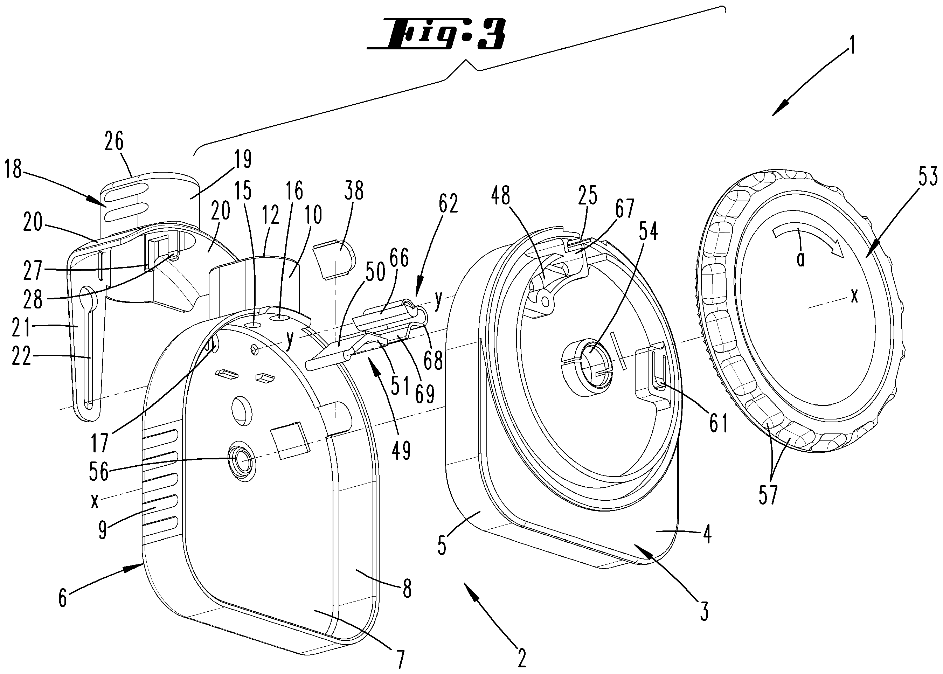

- Fig. 3: eine explosionsperspektivische Darstellung der Vorrichtung;

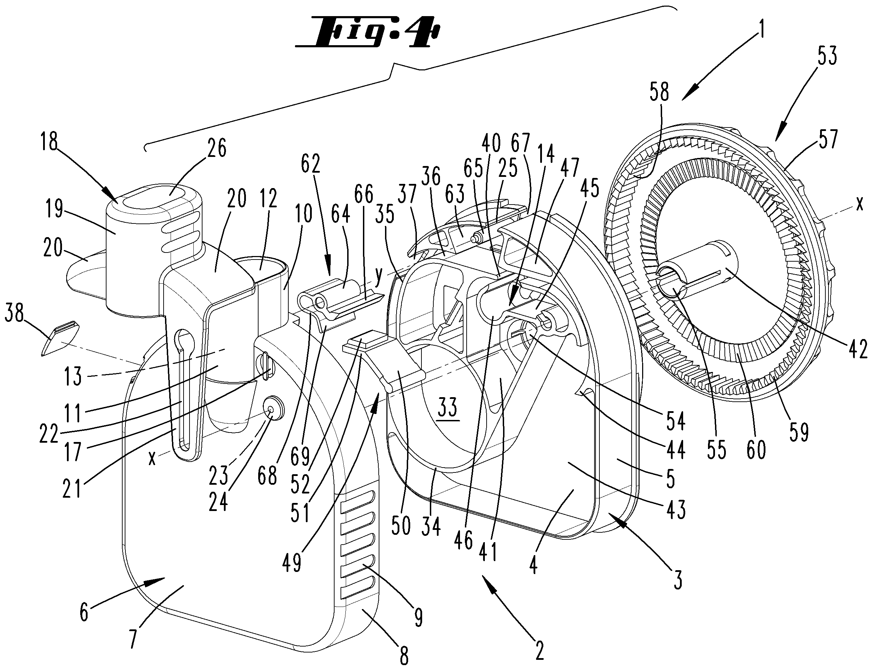

- Fig. 4: eine weitere explosionsperspektivische Darstellung der Vorrichtung;
- Fig. 5: die Vorrichtung in Ansicht bei von einem Mündungsstutzen abgehobener und verschwenkter Mündungs-Verschlusskappe;

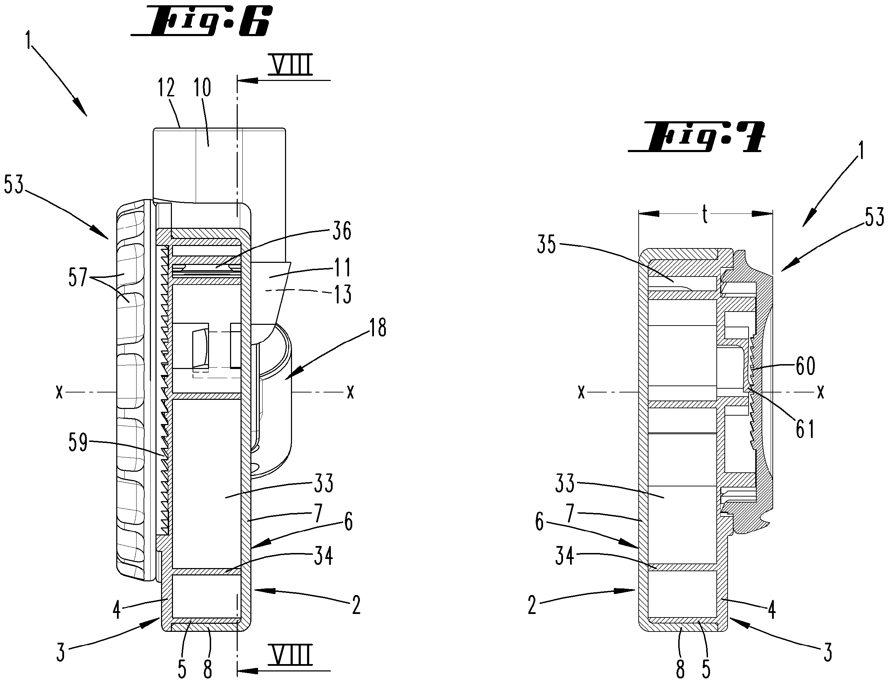

- Fig. 6: den Schnitt gemäß der Linie VI-VI in Fig. 5;
- Fig. 7: den Schnitt gemäß der Linie VII-VII in Fig. 5;
- Fig. 8: den Schnitt gemäß der Linie VIII-VIII in Fig. 6, mit in strichpunktierter Linienart wiedergegebener Verschlusskappe sowohl in der Verschlussstellung als auch in der den Mündungsstutzen freigebenden abgehobenen Stellung, weiter eine Grundstellung der Vorrichtung vor einer Erstbenutzung betreffend;
- Fig. 8a: eine der Fig. 8 entsprechende Darstellung, eine alternative Ausführungsform betreffend;
- Fig. 9: eine der Fig. 8 entsprechende Darstellung, jedoch im Zuge einer Betätigung der Drehhandhabe zur Verlagerung eines in der Vorrichtung vorgesehenen Bandstreifens;
- Fig. 10: eine Folgedarstellung zu Fig. 9;
- Fig. 11: eine weitere Folgedarstellung der Fig. 10, eine anschlagbegrenzte Verlagerungs-Endstellung betreffend;

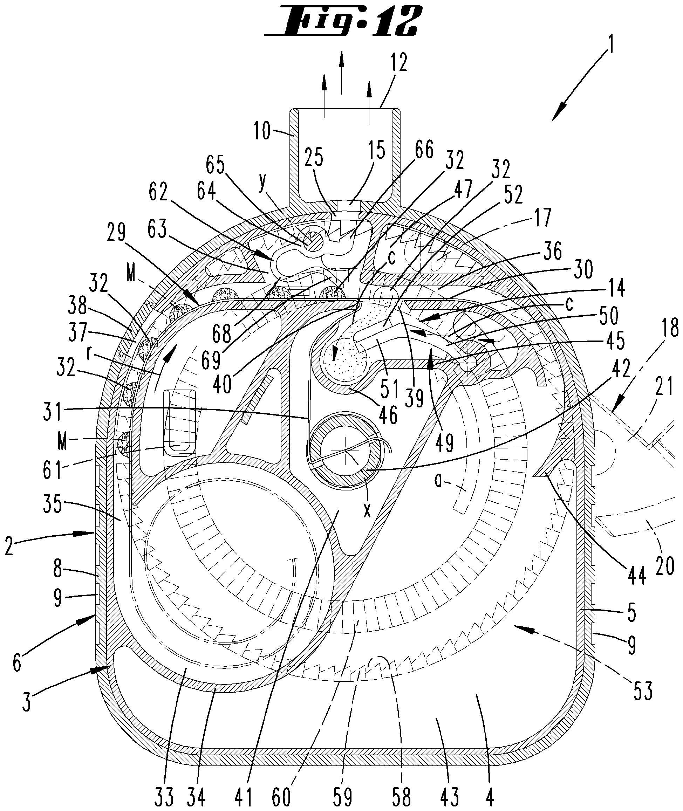

- Fig. 12: eine der Fig. 11 entsprechende Darstellung, jedoch im Zuge eines Inhalationsvorganges und damit einhergehendem Einsetzen einer Saugluftströmung zur Entleerung einer freigegebenen, ein Medikament bevorratenden Kammer;

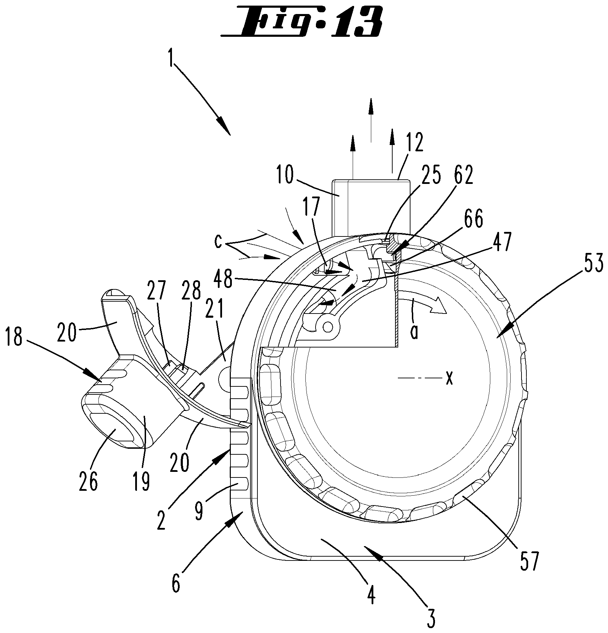

- Fig. 13: eine partiell geschnittene Perspektivdarstellung gemäß der Darstellung in Fig. 1, den Saugluftströmungsweg im Zuge der Inhalation darstellend;

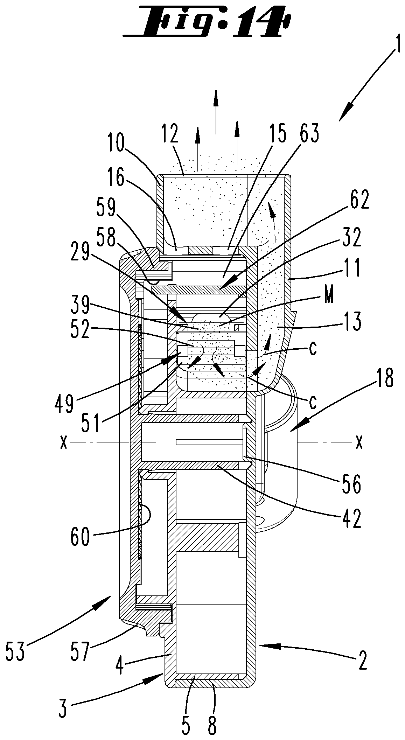

- Fig. 14: eine Vertikalschnittdarstellung durch die Vorrichtung zur weiteren Darstellung des Saugluftströmungsweges im Zuge der Inhalation;
- Fig. 15: eine der Fig. 11 entsprechende Darstellung, die Kappenaufsetzstellung betreffend;

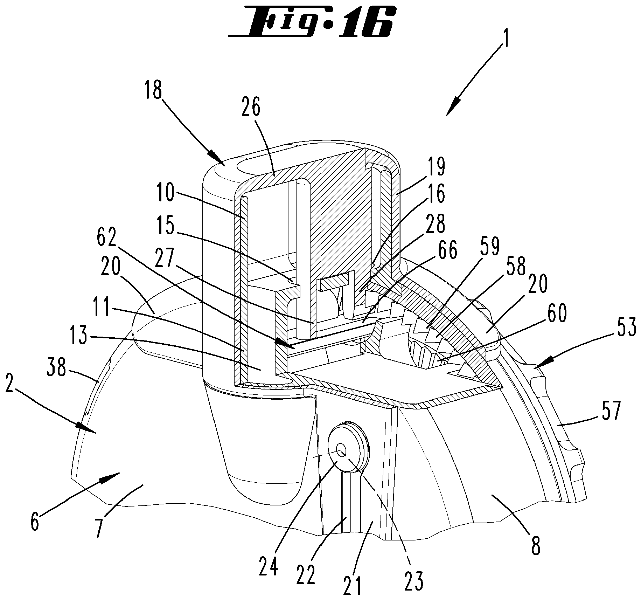

- Fig. 16: den Schnitt gemäß der Linie XVI-XVI in Fig. 15;

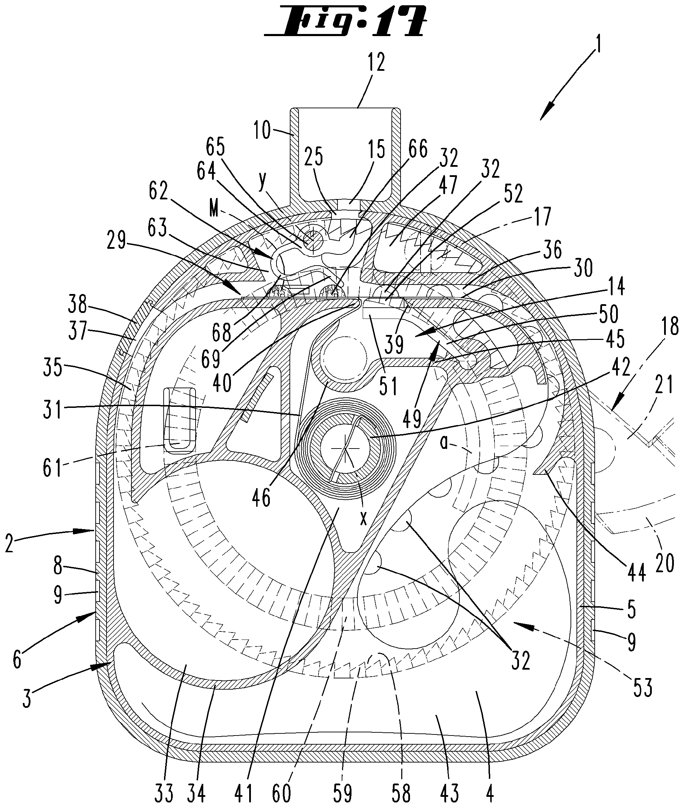

- Fig. 17: eine der Fig. 11 entsprechende Schnittdarstellung, betreffend eine Stellung nach Durchführung mehrerer portionierter Ausgaben von Medikamenten.

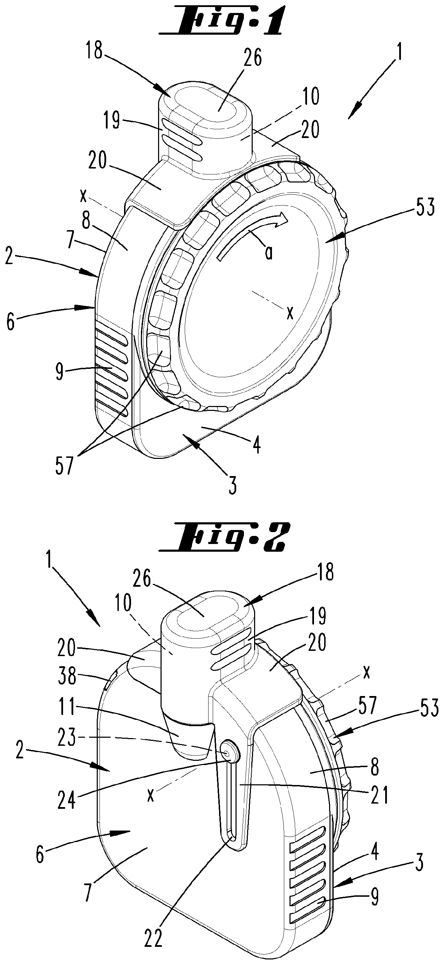

Dargestellt und beschrieben ist zunächst mit Bezug zu den Figuren 1 und 2 die Gesamt-Vorrichtung 1 zur portionierten Ausgabe von Medikamenten, insbesondere inhalationsfähigen, pulverförmigen Medikamenten.

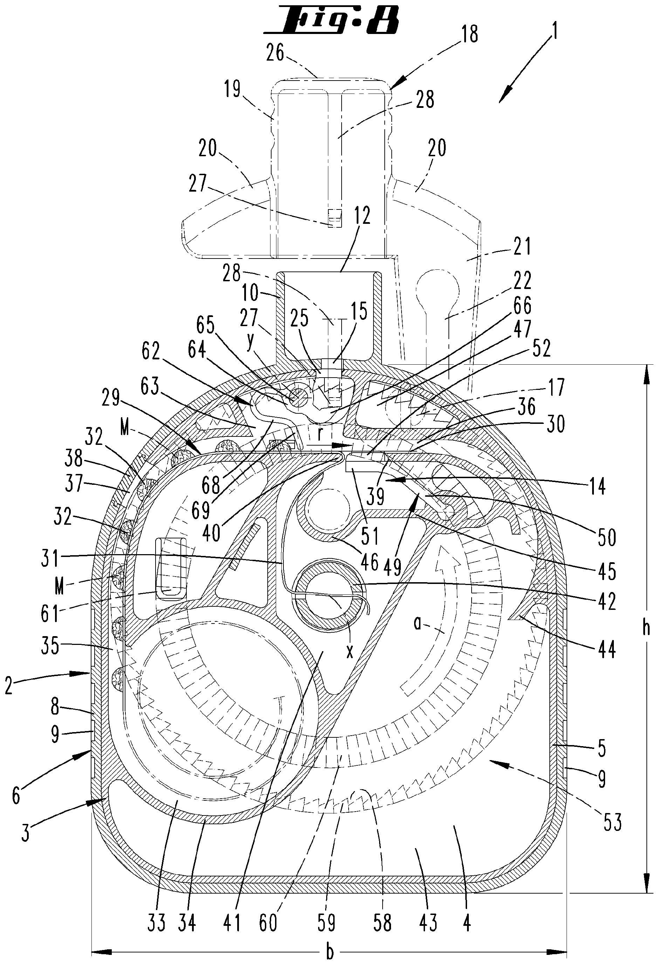

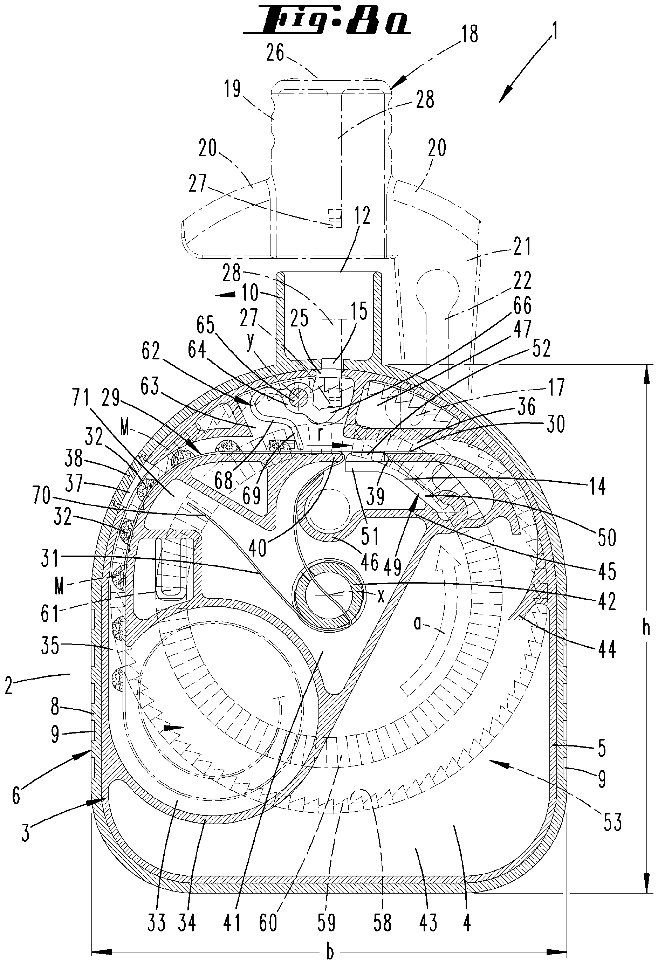

Die Vorrichtung 1 ist handbetätigbar durch den Benutzer, dies weiter bevorzugt bei handhabungsgünstigen Abmessungen der Vorrichtung. So ist eine bezogen auf einen Grundriss der Vorrichtung 1 gemäß der Darstellung in Fig. 8 betrachtete Breite b vorgesehen, die bevorzugt einem 0,7- bis 1,2-Fachen, weiter bevorzugt einem 0,9- bis 1-Fachen der senkrecht hierzu betrachteten Höhe h der Vorrichtung 1 (unter Nichtberücksichtigung eines Mundstückstutzens) entspricht. Die quer hierzu betrachtete Tiefe t der Vorrichtung 1 entspricht bevorzugt dem 0,15- bis 0,5-Fachen, weiter bevorzugt dem 0,2- bis 0,3-Fachen der Höhe h.

Entsprechend liegt die Vorrichtung 1 in einer Größe vor, die es erlaubt, diese körpernah, beispielsweise in Taschen von Kleidungsstücken, zu tragen.

Die Vorrichtung 1 weist zunächst ein Gehäuse 2 auf. Dieses ist im Wesentlichen zweiteilig gestaltet.

So ist zunächst bevorzugt ein schalenförmiges Gehäuseteil 3 vorgesehen, mit einem sich über die Breite b und die Höhe h erstreckenden Gehäuseboden 4 und einer im Wesentlichen entlang der Randkante des Gehäusebodens 4 sich senkrecht hierzu erstreckend umlaufenden Gehäusewandung 5.

Das Gehäuseteil 3 ist bevorzugt überfangen von einem, den zweiten Teil des Gehäuses 2 ausformenden Gehäusedeckels 6. Dieser weist eine bevorzugt parallel zum Gehäuseboden 4 des einen Gehäuseteiles 3 ausgerichtete Gehäusedecke 7 auf, von welcher umlaufend ausgehend sich senkrecht zur Gehäusedecke 7 erstreckend eine Deckelwandung 8 angeformt ist. Die Deckelwandung 8 umschließt umlaufend die Gehäusewandung 5 des Gehäuseteiles 3, wobei sich weiter bevorzugt die nach innen weisende Fläche der Deckelwandung 8 auf der freien, umlaufenden Randkante der Gehäusewandung 5 des Gehäuseteiles 3 abstützt.

Im Bereich der beiden sich in Höhenrichtung erstreckenden Deckelwandungsabschnitte sind wandungsaußenseitig durch regelmäßige Vertiefungen und Erhebungen ausgebildete Griffzonen 9 gebildet, die das sichere Greifen der Vorrichtung 1 insbesondere bei dessen Nutzung anbieten.

An dem Gehäusedeckel 6 ist weiter ein, eine - bezogen auf die Breite b - mittig das Gehäuse 2 durchsetzende Senkrechte mittig aufnehmender Mündungsstutzen 10 angeformt.

In einem, in Projektion auf den sich über die Breite b erstreckenden Gehäuseboden betrachteten Grundriss ist der Mündungsstutzen 10 so ausgebildet, dass sich in Breitenrichtung des Gehäuses 2 eine langlochartige Mündungsfläche ergibt. Hierbei erstreckt sich der Mündungsstutzen 10 im Wesentlichen ausgehend von dem Gehäuseboden 4 des einen Gehäuseteiles 3 bis über die Ebene der Gehäusedecke 7 des Gehäusedeckels 6, in welchem über die Gehäusedecke 7 überstehenden Bereich der Mündungsstutzen 10 übergeht in einen seitlich der Gehäusedecke 7 angeformten Kamin 11.

Ausgehend von den parallel gegenüberliegenden Abschnitten von Gehäusewandung 5 und Deckelwandung 8 erstrecken sich die Gehäusewandung 5 und Deckelwandung 8 bevorzugt kreisabschnittförmig, mit Anordnung des Mündungsstutzens 10 im Zenithbereich der Krümmung.

Die durch die Wandung des Mündungsstutzens 10 umfasste Mündungsöffnung 12 geht bevorzugt über in einen durch den Kamin 11 sowie dem zugewandten Wandungsabschnitt der Deckelwandung 8 begrenzten, im Wesentlichen parallel zu einer die Vorrichtung 1 durchsetzenden Senkrechten verlaufenden Strömungskanal 13. Dieser mündet bevorzugt fußseitig, d.h. der Mündungsöffnung 12 abgewandt, die Gehäusedecke 7 durchsetzend in einem in dem Gehäuse 2 ausgeformten Ausgabebereich 14.

Der durch die Deckelwandung 8 gebildete Boden des Mündungsstutzens 10 weist zwei in Breitenrichtung des Gehäuses 2 hintereinander und zueinander distanzierte Durchtrittsöffnungen 15 und 16 auf.

Des Weiteren ist in der Gehäusedecke 7 seitlich neben dem ausgeformten Kamin 11 eine die Gehäusedecke 7 durchsetzende Einströmöffnung 17 vorgesehen.

Bei Nichtgebrauch der Vorrichtung 1 ist der Mündungsstutzen 10 mit einer Mündungs-Verschlusskappe 18 überdeckt. Diese weist zunächst einen im Wesentlichen den Mündungsstutzen 10 und zumindest einen Teilabschnitt des Kamins 11 vollständig umfassenden, sowie die Mündungsöffnung 12 überspannenden, topfartigen Stülpabschnitt 19 auf, welcher in der Verschlussstellung der Vorrichtung gemäß Fig. 1 beidseitig übergeht in flügelartige Abschnitte 20 zur Abstützung auf der zugeordneten Oberfläche der Deckelwandung 8.

Von dem, in der Verschlussstellung der Einströmöffnung 17 in der Deckelwandung 8 zugewandten Abschnitt 20 erstreckt sich - bevorzugt in Parallelausrichtung zu einer Senkrechten durch das Gehäuse 2 - ein leistenartiger Führungsabschnitt 21, welcher zumindest annähernd flächig auf der zugewandten Oberfläche der Deckelwandung 8 aufliegt.

Der Führungsabschnitt 21 ist bevorzugt mit einer annähernd über die gesamte Länge desselben sich erstreckenden, schlitzartigen Ausnehmung 22 versehen. In diese greift ein sich senkrecht von der Deckelwandung 8 erhebender, bevorzugt mit der Deckelwandung 8 materialeinheitlich einstückig ausgebildeter Zapfen 23, welcher sich gegenüberliegend zum Wurzelbereich in der Gehäusedecke 7 in einen radial vergrößerten Kragenabschnitt 24 erweitert, zum Übergriff über die parallel verlaufenden Randkanten der Ausnehmung 22.

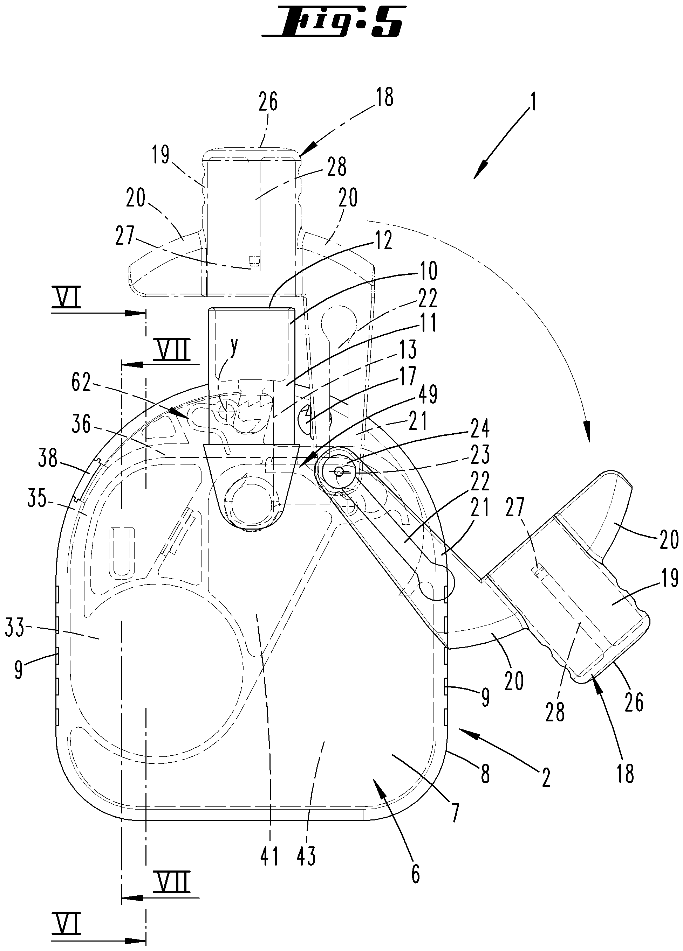

Zufolge dieser Ausgestaltung ist die Mündungs-Verschlusskappe 18 verliersicher an dem Gehäuse 2 gehaltert, hierbei längsverschieblich in Aufsetz- bzw. Abziehrichtung der Verschlusskappe mit Bezug auf den Mündungsstutzen 10 sowie schwenkbar um den Zapfen 23 in eine zum Mündungsstutzen 10 verschwenkte, seitliche Stellung gemäß Fig. 5. In dieser abgeschwenkten Stellung stützt sich die Mündungs-Verschlusskappe 18 mit einem Randbereich des dem Führungsabschnitt 21 zugewandten Abschnittes 20 bevorzugt an der zugewandten Fläche der Deckelwandung 8 ab.

Ausgehend von einer in Verschlussstellung die Mündungsöffnung 12 überspannenden Kappendecke 26 des Stülpabschnittes 19 erstrecken sich im Wesentlichen senkrecht nach unten zwei Vorsprünge 27, 28, welche derart positioniert und hinsichtlich ihrer Länge gewählt sind, dass diese durch die Durchtrittsöffnungen 15 und 16 im Bereich des Bodens des Mündungsstutzens 10 treten.

Die Gehäusewandung 5 des Gehäuseteiles 3 ist unterhalb der Durchtrittsöffnungen 15 im Bereich der Deckelwandung 8 unterbrochen, zur Ausbildung eines Durchgreifschlitzes 25.

In dem Gehäuse 2 ist ein Bandstreifen 29 aufgenommen. Hierbei handelt es sich bevorzugt um einen langgestreckten Streifen mit einer Länge, die dem 20-bis 40-Fachen, weiter bevorzugt dem 25- bis 35-Fachen der senkrecht hierzu betrachteten Breite des Streifens entspricht. In bevorzugter Ausgestaltung weist der Bandstreifen 29 eine Breite von 10 bis 15 mm, weiter bevorzugt 12 bis 13 mm und eine senkrecht hierzu betrachtete Länge von 300 bis 500 mm, weiter bevorzugt etwa 400 mm auf.

Der Bandstreifen 29 ist derart in dem Gehäuse 2 eingelegt, dass sich dieser mit seiner Breite in Breitenrichtung des Gehäuses 2 erstreckt.

Der Bandstreifen 29 besteht tim Wesentlichen aus gleich breiten, übereinander liegenden Basisstreifen 30 und Verschlussstreifen 31, wobei der Basisstreifen 30 bevorzugt ein Metallstreifen ist, während der Verschlussstreifen 31 bevorzugt kunststofffolien- und/oder aluminiumfolienartig ausgebildet ist.

Aus dem Basisstreifen 30 heraus sind abgewandt zum Verschlussstreifen 31 sich erhebende in Figur 8 in Richtung der Mundstücköffnung 12 weisende Kammern 32 ausgeformt. Diese sind weiter bevorzugt aus dem Material des Basisstreifens 30 herausgedrückt. Jede Kammer 32 weist hierbei eine Breite auf, die bevorzugt dem 0,5- bis 0,9-Fachen der Bandbreite entspricht, weiter bevorzugt etwa dem 0,75-Fachen.

In bevorzugter Ausgestaltung ist eine Kammerbreite von 6 bis 10 mm, weiter bevorzugt 8 mm vorgesehen. Die senkrecht hierzu betrachtete Länge einer jeden Kammer 32 - in Längserstreckung des Bandstreifens 29 - entspricht bevorzugt dem 0,3- bis 0,7-Fachen, weiter bevorzugt etwa dem 0,5-Fachen der Kammerbreite.

Alle Kammern 32 weisen dasselbe Aufnahmevolumen auf und sind bevorzugt mit einem pulverförmigen, inhalierfähigen Medikament M befüllt.

In Längserstreckungsrichtung des Bandstreifens 29 sind die Kammern 32 gleichmäßig zueinander beabstandet angeordnet, wobei bevorzugt über die Länge des Bandstreifens 29 40 bis 100, bevorzugt 50 bis 70, insbesondere etwa 60 Kammern 32 vorgesehen sind.

Der Abstand zweier hintereinander liegender Kammern 32 - mit Bezug auf die Längserstreckung des Bandstreifens 29 - entspricht etwa der gewählten Kammerbreite, weiter bevorzugt 6 bis 10 mm, insbesondere etwa 8 mm, weiter bevorzugt entsprechend dem 0,5- bis 0,9-Fachen der Bandbreite, bevorzugt etwa der 0,75-Fachen Bandbreite.

Der Bandstreifen 29 ist in einer zwischen dem Gehäuseboden 4 und der Deckelwandung 8 ausgebildeten Wickelkammer 33 lose aufgewickelt eingelegt. Die Wickelkammer 33 ist im Grundriss im Wesentlichen kreisförmig, wobei die die Wickelkammer 33 umgebende Wandung 34 bevorzugt einstückig und materialeinheitlich mit dem Gehäuseteil 3 ausgeformt ist. Die Wickelkammerwandung 34 erstreckt sich bevorzugt senkrecht zum Gehäuseboden 4.

Weiter bevorzugt ist die Wickelkammer 33 insbesondere dem geradlinig verlaufenden Abschnitt der Gehäusewandung 5 zugeordnet, der der Einströmöffnung 17 abgewandt ist. Das Wickelkammerinnere öffnet sich bevorzugt in eine im Wesentlichen tangential austretende Schlitzführung 35, welche bevorzugt parallel verlaufend zur zugewandten Gehäusewandung 5 in einen quergerichteten - in einer gedachten Verlängerung den kreisabschnittförmigen Bereich der Gehäusewandung 5 sekantenartig schneidenden - schlitzartigen Führungsabschnitt 36 übergeht.

Dieser Führungsabschnitt 36 erstreckt sich bevorzugt parallel zu einem die parallel zueinander ausgerichteten Abschnitte der Gehäusewandung 5 verbindenden Gehäusewandungsabschnitt. Der Übergang von der senkrechten Schlitzführung 35 in den hierzu betrachtet waagerechten Führungsabschnitt 36 ist bevorzugt derart verrundet, dass der über diesen Bereich geführte Bandstreifen 29 insbesondere knickfrei, darüber hinaus aber auch bevorzugt reibungsarm geführt werden kann.

Der Bandstreifen 29 liegt bevorzugt derart in der Schlitzführung 35 und dem Führungsabschnitt 36 ein, dass die Erhebungen der Kammern 32 stets nach außen, d.h. im Wesentlichen in Richtung auf die Gehäusewandung 5 weisen, wobei weiter der Abstand der die Schlitzführung 35 und den Führungsabschnitt 36 begrenzenden, im Wesentlichen parallel zueinander verlaufenden Wandungen angepasst ist an die senkrecht zur Breiten- und Längserstreckung des Bandstreifens 29 betrachtete Höhe des Bandstreifens 29, so dass hierdurch eine exakte, wenngleich auch bevorzugt reibungsarme Führung des Bandstreifens 29 gegeben ist.

Bevorzugt zugeordnet dem verrundeten Übergangsbereich von Schlitzführung 35 zum Führungsabschnitt 36 ist in dem zugeordneten Abschnitt der Gehäusewandung 5 ein fensterartiger Ausbruch 37 vorgesehen, der im Bereich der Deckelwandung 8 überdeckt ist von einem in der Deckelwandung 8 ausgebildeten Transparentbereich 38. Durch das so gebildete transparente Fenster ist dem Benutzer ein Blick auf den in der Schlitzführung 35 geführten Bandstreifen 29 angeboten, insbesondere zur Erfassung einer bevorzugt numerischen Information.

So ist bevorzugt jeder Kammer 32 auf der, der Erhebung zugewandten Seite des Basisstreifens 30 eine Ziffer zugeordnet, die den Benutzer darüber informiert, wie viele gefüllte Kammern 32 noch zur Ausgabe des Medikaments M zur Verfügung stehen oder alternativ wie viele Kammern 32 bereits zufolge Ausgabe des Medikaments M genutzt wurden.

Im Wesentlichen senkrecht unterhalb des Durchgreifschlitzes 25 in der Gehäusewandung 5 ist die, den Führungsstreifen 36 verschlussstreifenseitig begrenzende Wandung unterbrochen, zur Ausbildung einer Durchfallöffnung 39.

Die, ausgehend von der Schlitzführung 35 erste Randkante der Durchfallöffnung 39 formt eine Umlenkkante 40, um welche der nunmehr von dem Basisstreifen 30 des Bandstreifens 29 gelöste Verschlussstreifen 31 geführt ist.

Die Führung ist hierbei so gewählt, dass ein scharfes Abknicken des Verschlussstreifens 31 erreicht ist, dies bevorzugt unter Einschluss eines spitzen Winkels zwischen dem Bereich des Verschlussstreifens 31 der vor der Umlenkkante 40 noch an dem Basisstreifen 30 verhaftet ist und dem unmittelbar hinter der Umlenkkante 40 geführten Abschnitt des Verschlussstreifens 31 von etwa 15 bis 45°, weiter etwa 30°.

Der von dem Basisstreifen 30 gelöste Verschlussstreifen 31 ist geführt in einen Verschlussstreifen-Wickelraum 41. Das diesbezügliche freie Ende des Verschlussstreifens 31 ist bevorzugt festgelegt an einem drehantreibbaren Dorn 42, um welchen Dorn 42 im Zuge des drehenden Antriebs desselben der Verschlussstreifen 31 nach und nach aufgewickelt wird. Die Aufwickelrichtung (Pfeil a) ist entgegengesetzt gerichtet zu der Abwickelrichtung des Bandstreifens 29 in der Wickelkammer 33.

Die Drehachse x des Dorns 42 bildet hierbei bevorzugt zugleich die Radiusachse für den oberen, verrundeten Bereich von Gehäusewandung 5 und Deckelwandung 8, wobei weiter auf einer den Durchgreifschlitz 25 durchsetzenden Radiuslinie - ausgehend von der Drehachse x - die Umlenkkante 40 und die sich hieran anschließende Durchfallöffnung 39 angeordnet sind.

Der Verschlussstreifen-Wickelraum 41 erstreckt sich seitlich und oberhalb der Wickelkammer 33.

In Fig. 8a ist eine alternative Ausgestaltung insbesondere hinsichtlich des Gehäusequerschnitts, weiter insbesondere hinsichtlich der Führung des Verschlussstreifens 31 in der Bestückungsstellung der Vorrichtung 1 dargestellt. Ein über die erste Kammer 32 in Bewegungsrichtung r hinausgehender Festlegungsabschnitt des Verschlussstreifens 31 ist, einen bevorzugt diametral zur Drehachse ausgerichteten Schlitz des Dornes 42 durchsetzend, so eingelegt, dass dessen fahnenartiges freies Ende 70 in eine sich zwischen der Wickelkammer 33 und dem Verschlussstreifen-Wickelraum 41 ergebende Kammer 71 erstreckt. Im Zuge der Drehhandhabenbetätigung, gegebenenfalls über mehrere Inhalationsvorgänge hinweg, wird der freie Fahnenabschnitt des Verschlussstreifens 31 sukzessive aus der Kammer 71 herausgezogen, dies unter zunächst doppellagiger Einwicklung des fahnenartigen Abschnittes beziehungsweise nachfolgender Überwicklung des fahnenartigen Abschnittes durch nachgezogene Bereiche des Verschlussstreifens 31.

Der von dem Verschlussstreifen 31 befreite Basisstreifen 30 ist in Verlängerung des Führungsabschnittes 36 geführt, hiernach bevorzugt übergehend in einen kreisabschnittförmig nach unten und zur Gehäusemitte hin tendierenden kreisabschnittförmigen Führungsbereich, welch letzterer frei in einer Einschubkammer 43 mündet.

Diese Einschubkammer 43 erstreckt sich bevorzugt sowohl seitlich des Verschlussstreifen-Wickelraumes 41 als auch seitlich sowie partiell unterhalb der Wickelkammer 33. Entsprechend ist bevorzugt der Dorn 42 sowie der den Dorn 42 aufnehmende Verschlussstreifen-Wickelraum 41 im Wesentlichen zwischen der Wickelkammer 33 und der Einschubkammer 43 angeordnet.

Der Basisstreifen 30 wird in die Einschubkammer 43 eingeschoben, wobei ein endseitiger, bevorzugt schanzenartiger Einführabschnitt 44 ein Einschieben des Basisstreifens 30 tendenziell zur Gehäusemitte hervorruft.

Mit zunehmender Länge des in die Einschubkammer 43 eingeschobenen Bandstreifens 29 legt sich dieser bevorzugt stapelartig in die Einschubkammer 43 ein.

Die Durchfallöffnung 39 geht unterhalb des Führungsabschnittes 36 über in den Ausgabebereich 14, welcher im Wesentlichen umfasst ist von einer senkrecht zur Ebene des Gehäusebodens 4 sich erstreckenden Wandung 45. Ein Teilbereich der Wandung 45 ist bevorzugt ersetzt durch den zwischen der Umlenkkante 40 und der Wandung 45 geführten und bevorzugt gespannten Abschnitt des Verschlussstreifens 31. Weiter ist die Wandung 45 - wie vorbeschrieben - von der Durchfallöffnung 39 durchsetzt.

Im Bodenbereich der Wandung 45, zugewandt dem wandungsaußenseitig an der Wandung 45 entlanggeführten Verschlussstreifen 31, formt die Wandung 45 bevorzugt einen trogartigen, im Wesentlichen sich nach oben in Richtung auf die Durchfallöffnung 39 öffnenden Abschnitt 46 aus. Bevorzugt mündet in diesen trogartigen Abschnitt der Strömungskanal 13 des Kamins 11.

Bevorzugt oberhalb des Ausgabebereiches 14, weiter oberhalb des Führungsabschnittes 36 ist eine Einströmkammer 47 vorgesehen, in die die Einströmöffnung 17 im Bereich der Deckelwandung 8 mündet.

Die Einströmkammer 47 ist bevorzugt über einen in Breitenrichtung des Gehäuses 2 ebenenversetzt ausgebildeten Strömungskanalabschnitt 48 mit dem Ausgabebereich 14 strömungsmäßig verbunden. Der Strömungskanalabschnitt 48 mündet bevorzugt in einem, dem trogartigen Abschnitt 46 des Ausgabebereiches 14 abgewandten Endbereich.

Der den Ausgabebereich 14 bildende Raum ist durch eine im Wesentlichen sich in diesem Raum diagonal erstreckende Klappe 49 geteilt, so insbesondere in einer Klappenverschlussstellung zur Trennung des mit dem Strömungskanalabschnitt 48 verbundenen Bereiches und des sich unterhalb der Durchfallöffnung 39 erstreckenden Bereiches.

Die Klappe 49 ist bevorzugt dichtlappenartig gestaltet, weiter bevorzugt aus einem gummiartigen, elastisch rückstellfähigen Material hergestellt.

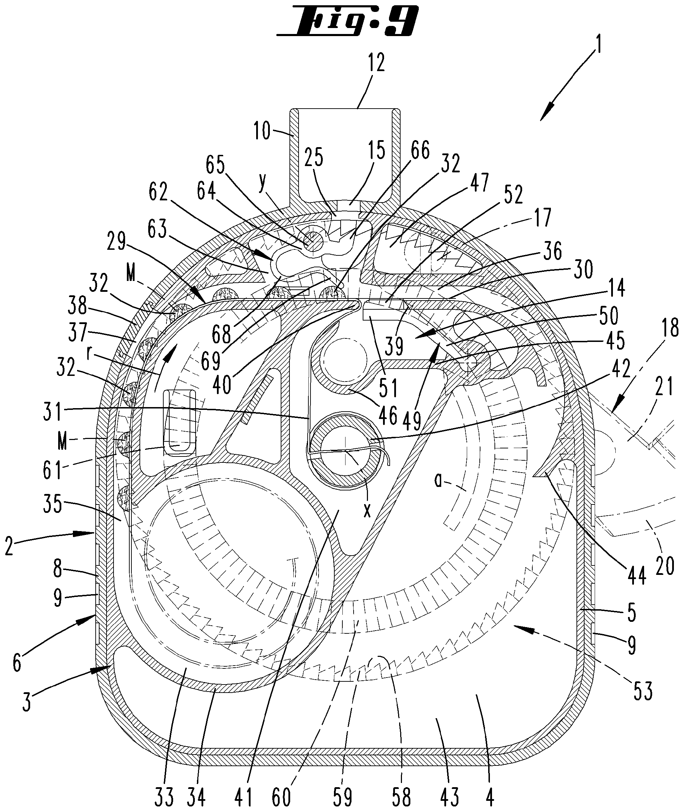

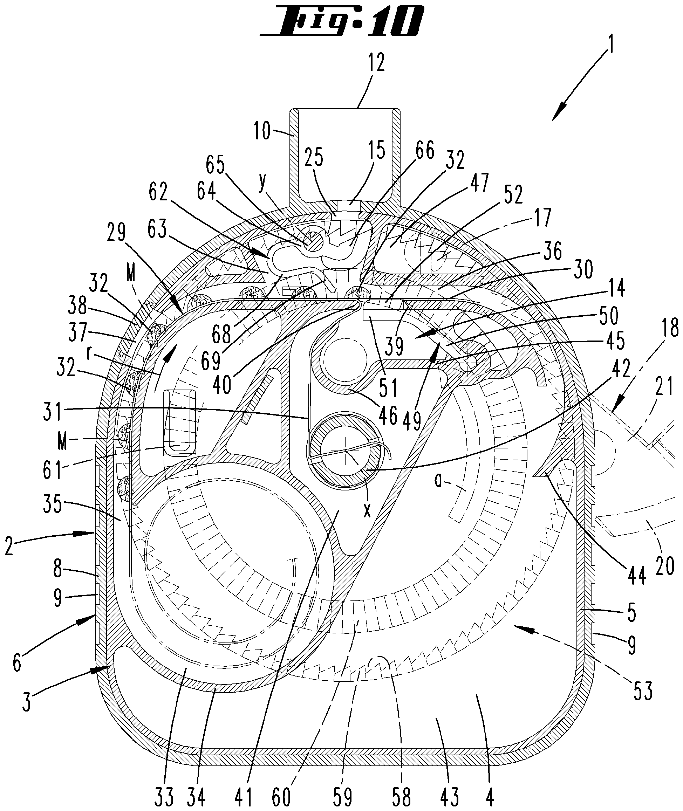

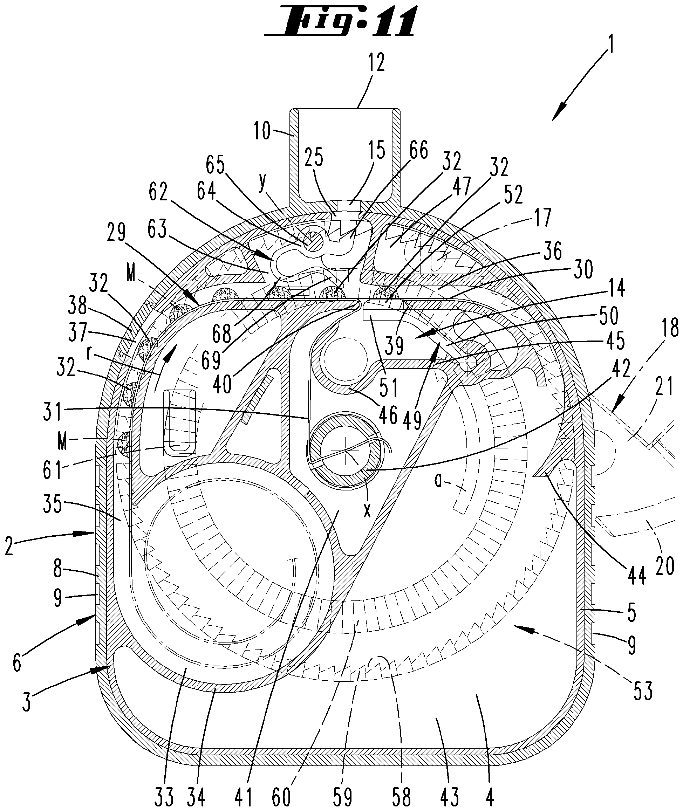

Die Klappe 49 weist im Wesentlichen einen elastisch ausbiegbaren Arm 50, welcher sich weiter im Wesentlichen mit Bezug auf eine das Gehäuse 2 und den Mündungsstutzen 10 durchsetzende Senkrechte in einer unbeeinflussten Grundstellung gemäß Figuren 8 bis 11 in einem spitzen Winkel von etwa 45° erstreckt.

Bevorzugt fußseitig ist der Arm 50 gefesselt im Bereich der bodenseitigen Wandung 45 im Ausgabebereich 14. Diesem Fesselungsbereich gegenüberliegend ist an dem Arm 50 ein Dichtabschnitt 51 angeformt. Dieser erstreckt sich in einem Vertikalschnitt in einem stumpfen Winkel zu dem Arm 50, weiter bevorzugt in der unbeeinflussten Grundstellung bevorzugt parallel zur bodenseitigen Wandung 45 des Ausgabebereiches 14.

Der Dichtabschnitt 51 trägt bevorzugt einen mit Bezug auf die Darstellungen in den Figuren 8 bis 11 nach oben abragenden Eingriffsfortsatz 52, zum Eingriff in der unbeeinflussten Stellung der Klappe 49 in die Durchfallöffnung 39.

Die Klappe 49 erstreckt sich mit Bezug senkrecht zur Betrachtungsebene in den Figuren 8 bis 11 bevorzugt über die gesamte Breite des Ausgabebereiches 14.

Die weiter mit Bezug auf die Darstellungen betrachtete vertikale Dicke des Eingriffsfortsatzes 52 entspricht bevorzugt der Materialstärke der die Durchfallöffnung 39 begrenzenden Wandung des Führungsabschnittes 36.

Teil der Vorrichtung 1 ist weiter eine Drehhandhabe 53. Hierbei handelt es sich bevorzugt um ein scheiben- oder tellerartiges Element mit kreisförmigem Umfang. Die Drehhandhabe 53 ist bevorzugt der nach außen weisenden Fläche des Gehäusebodens 4 zugeordnet angeordnet.

Zentral, d.h. die Drehachse der Drehhandhabe 53 aufnehmend, ist wandungsinnenseitig der Drehhandhabe 53 der Dorn 42 angeformt, welcher den Gehäuseboden 4 im Bereich einer Öffnung 54 durchsetzend in das Gehäuseinnere eintaucht und hier, wie vorbeschrieben, den Verschlussstreifen-Wickelraum 41 durchsetzt. Eine Führung erfährt hierbei der Dorn 42 und über diesen entsprechend auch die Drehhandhabe 53 bevorzugt über einen gehäuseinnenseitig der Gehäusedecke 7 angeformten und in eine zugewandte Stirnöffnung 55 des Dornes 42 eintauchenden Zapfen 56.

Die Drehhandhabe 53 weist bevorzugt einen an den Krümmungsradius der Gehäusewandung 5 beziehungsweise Deckelwandung 8 im Bereich des Mündungsstutzens 10 angepassten Radius auf.

Der umlaufende Randbereich der Drehhandhabe 53 ist handhabungsgünstig gestaltet zufolge Ausbildung gleichmäßig über den Umfang vorgesehener Greifmulden 57.

Die Drehhandhabe 53 ist im Wesentlichen ein Teil eines Schrittschaltwerkes zur schrittweisen Weiterführung des Bandstreifens 29. Hierzu ist zunächst wandungsinnenseitig des umlaufenden, die Greifmulden 57 aufweisenden Randes der Drehhandhabe 53 ein bevorzugt nach radial innen ausgerichteter, umlaufender Zahnkranz 58 vorgesehen. Die Zähne des Zahnkranzes 58 sind in der vorgegebenen Drehrichtung der Drehhandhabe 53 gemäß Pfeil a bevorzugt sägezahnartig angestellt.

Der Zahnkranz 58 setzt sich bevorzugt fort in einen auf der dem Gehäuseboden 4 zugewandten Fläche der Drehhandhabe 53 angeformten Zahnring 59, dessen Zähne entsprechend bevorzugt in Axialrichtung der Drehhandhabe 53 weisen.

Konzentrisch zum Zahnkranz 58 ist innenseitig der Drehhandhabe 53, d.h. auf der dem Gehäuseboden 4 zugewandten Handhabenfläche bevorzugt ein weiterer, gegenüber dem Zahnkranz 58 durchmesserverrigerter Zahnring 60 eingearbeitet. Dieser Zahnring 60 ist im Wesentlichen in Art einer Hirth-Verzahnung ausgeformt.

In den Zahnring 60 greift ein bevorzugt im Bereich des Gehäusebodens 4 ausgeformter, federnd rückstellfähig ausgebildeter Sperrzahn 61 ein. Sein Zusammenwirkungsbereich mit dem Zahnring 60 ist so ausgebildet, dass dieser lediglich eine Drehung der Drehhandhabe 53 in die vorgegebene Drehrichtung gemäß Pfeil a zulässt, eine Drehung der Drehhandhabe 53 entgegen Pfeilrichtung a jedoch zufolge Sperrung unterbindet.

Der Zahnkranz 58 dient bevorzugt zur Zusammenwirkung mit einem in dem Gehäuse 2 um eine parallel zur Drehachse x ausgerichteten Schwenkachse y schwenkbar gelagerten Sperrglied 62.

Dieses Sperrglied 62 ist bevorzugt in einer mit Bezug auf die Darstellungen oberhalb des Führungsabschnittes 36, in Bewegungsrichtung r des Bandstreifens 29 im Wesentlichen vor der Durchfallöffnung 39 ausgeformten Sperrglied-Kammer 63 angeordnet, dies weiter im Wesentlichen unterhalb des Mündungsstutzens 10.

In diese Sperrglied-Kammer 63 mündet zugleich die anderenends sich zum Mündungsstutzen 10 hin öffnende Öffnung 15.

Das Sperrglied 62 weist bevorzugt zunächst eine Nabe 64 auf, welche auf einem bevorzugt an dem Gehäuseteil 3 wandungsinnenseitig des Gehäusebodens 4 angeformten Achskörper 65 schwenkbeweglich sitzt.

Von dieser Nabe 64 ragt im Wesentlichen nach radial außen ein Arm ab, der endseitig einen in den Darstellungen nach oben tendenziell in Richtung auf den Mündungsstutzen 10 weisenden Eingriffszahn 66 trägt.

Bevorzugt ist der Eingriffszahn 66 senkrecht zur Betrachtungsebene beispielsweise der Fig. 8 über die Breite des Gehäuses 2 in Richtung auf die Drehhandhabe 53 verlängert, hierbei einen fensterartigen Durchbruch 67 in dem Gehäuseboden 4 durchsetzend, zum Eingriff in den Zahnring 59 der Drehhandhabe 53.

Des Weiteren ist an der Nabe 64 bevorzugt im Wesentlichen diametral gegenüberliegend zu dem den Eingriffszahn 66 tragenden Arm ein geschwungener Federarm 68 angeformt, dessen freies, nach unten in Richtung auf den Bandstreifen 29 weisende Ende einen reinen Abtastfinger 69 ausbildet.

Die Funktion der Vorrichtung 1 ist wie folgt:
In der in Fig. 8 dargestellten Situation vor einer ersten Nutzung der Vorrichtung 1 ist der Bandstreifen 29 derart in dem Gehäuse 2 eingelegt, dass ausgehend von dem Bandstreifen-Wickel in der Wickelkammer 33 sich der Bandstreifen über die Schlitzführung 35 und den Führungsabschnitt 36 zumindest bis in Überdeckung zu der Sperrglied-Kammer 63 erstreckt.

Ein über die in Bewegungsrichtung r erste Kammer 32 des Basisstreifens 29 hinausragender Abschnitt des Verschlussstreifens 31 ist um die Umlenkkante 40 gelegt und an dem Dorn 42 festgelegt, während sich ein diesbezüglicher Abschnitt des Basisstreifens 30 im Wesentlichen in Verlängerung der Erstreckung des Bandstreifens im Führungsabschnitt 36, die Durchfallöffnung 39 überbrückend, weiter in Richtung auf die Einschubkammer 43 erstreckt.

Zur Vorbereitung insbesondere eines Inhalationsvorganges wird zunächst von dem Mündungsstutzen 10 die Mündungs-Verschlusskappe 18 abgezogen und hiernach bevorzugt zufolge Verschwenken seitlich an dem Gehäuse 2 abgelegt.

Über die Drehhandhabe 53 wird hiernach der Dorn 42 in der vorgegebenen Drehrichtung gemäß Pfeil a gedreht.

Der im Zuge der Drehung zunächst in den in Bewegungsrichtung r betrachteten Zwischenraum zwischen zwei hintereinanderliegenden Kammern 32 eingreifende Abtastfinger 69 des Sperrgliedes 62 wird im Zuge der weiteren Drehverlagerung und damit einhergehender Verlagerung des Bandstreifens 29 insbesondere in dem Führungsabschnitt 36 - dies zufolge einer Zugverlagerung über den um den Dorn 42 sich aufwickelnden Verschlussstreifen 31 - durch die nächstfolgende erhabene Kammer 32 beaufschlagt. Dies führt zufolge der entsprechenden Abstützung des Abtastfingers 69 an der zugeordneten Kammer 32 zu einer Schwenkverlagerung des gesamten Sperrgliedes 62 bis in eine Stellung, in welcher der Eingriffszahn 66 des Sperrgliedes 62 in den Zahnkranz 58 der Drehhandhabe 53 sperrend eingreift. Die Drehhandhabe 53 ist entsprechend in dieser Stellung nicht weiter drehverlagerbar. Die Drehverlagerung in vorgegebener Richtung gemäß Pfeil a ist durch das Sperrglied 62 gesperrt praktisch ohne Drehhaft-Kraftbelastung der Kammer 32. Die Drehverlagerung in entgegengesetzter Richtung durch den Sperrzahn 61.

Die Sperrung der Drehhandhabe 53 gibt dem Benutzer das Signal, dass die Vorrichtung 1 nunmehr bereit ist, zur portionierten Ausgabe des Medikaments M, insbesondere zur Inhalation desselben.

Die in Bewegungsrichtung r der mit dem Abtastfinger 69 des Sperrgliedes 62 zusammenwirkenden Kammer 32 vorgeordnete Kammer 32 liegt in dieser Stellung oberhalb der Durchfallöffnung 39, ist darüber hinaus zufolge Abziehen um die Umlenkkante 40 von dem Verschlussstreifen 31 befreit. Entsprechend ist die der Durchfallöffnung 39 zugeordnete Kammer 32 nach unten - bezogen auf die Darstellungen - geöffnet.

Das Medikament M in dieser Kammer 32 ist jedoch gegen Herausrieseln zunächst gesichert durch die in Schließstellung verweilende Klappe 49, in welcher deren Eingriffsfortsatz 52 in die Durchfallöffnung 39 eingreifend die zugeordnete Kammer 32 zunächst verschließt.

Dies verhindert zugleich auch eine Doppeldosierung in dem Falle, dass der Benutzer sich in dieser Vorrichtungsstellung dazu entschließt, den Inhalationsvorgang nicht vorzunehmen. Ein Zurücksetzen und Wiederaktivieren des Systems - wie nachstehend näher beschrieben - führt zwar zur entsprechenden Bereitstellung der nachfolgenden Kammer 32. Die zuvor geöffnete und bereitgestellte Kammer 32 wird hierbei, das Medikament M weiterhin beinhaltend, in Richtung auf die Einschubkammer 43 verlagert, wobei im Zuge dieser weiteren Einschubverlagerung die Kammeröffnung unterseitig durch die zugewandte Wandung des Führungsabschnittes 36 überdeckt ist. Ein Ausrieseln des in der geöffneten Kammer 32 einliegenden Medikaments M wird bevorzugt erst im Bereich der Einschubkammer 43 erreicht, somit entsprechend in einem nicht zum Zwecke der Inhalation dienenden Strömungsbereich.

Zum Zwecke der Inhalation wird der Mündungsstutzen 10 durch die Lippen des Benutzers umschlossen. Zufolge tiefen Einatmens wird eine Saugluftströmung (Pfeile c) erzeugt, die zunächst in dem, dem Abschnitt 46 des Ausgabebereiches 14 zugeordneten Bereich einen Unterdruck erzeugt, der zu einem Einsetzen einer Verschwenkung der Klappe 49 in eine, die Durchfallöffnung 39 freigebende Stellung führt, dies entgegen der Rückstellfederkraft der Klappe 49.

Mit Einschwenken der Klappe 49 wird der Strömungsweg zu dem Strömungskanal-Abschnitt 48 freigegeben, woraufhin mit der dann einsetzenden, über die Einströmöffnung 17 angesaugten Saugluft die Klappe 49 gegebenenfalls weiter entgegen ihrer Rückstellkraft verlagert wird.

Durch die verlagerte Klappe 49 ist die Öffnung der, der Durchfallöffnung 39 zugeordneten, verschlussstreifenfreien Kammer 32 zur Ausgabe des Medikaments M freigelegt. Dieses wird, gegebenenfalls in Überlagerung von einem Ausrieseln aus der Kammer 32, durch die Saugluft aus der Kammer 32 ausgeräumt, weiter bevorzugt insbesondere im Bereich des Abschnittes 46 des Ausgabebereiches 14 zur bevorzugten gleichmäßigen Verteilung in der Saugluft verwirbelt und hiernach über den Strömungskanal 13, den Kamin 11 und den Mündungsstutzen 10 zur Inhalation ausgebracht.

Mit Beendigung des Inhalationsvorganges und somit dem Abbrechen der Saugluftströmung fällt die Klappe 49 selbsttätig zufolge der gegebenen Rückstellkraft wieder in die, die Durchfallöffnung 39 verschließende Stellung zurück.

Zum Abschluss wird die Mündungs-Verschlusskappe 18 auf den Mündungsstutzen 10 aufgesetzt, wobei die kappenseitigen Vorsprünge 27 und 28 die entsprechenden Durchtrittsöffnungen 15 und 16 durchsetzen, wobei weiter insbesondere mittels des Führungsabschnittes 21 der Mündungs-Verschlusskappe 18 die Einströmöffnung 17 überdeckt ist, dies insbesondere zur Verhinderung des Eintretens von Schmutz oder dergleichen bei Nichtbenutzung der Vorrichtung 1.

Der die Durchtrittsöffnung 15 durchsetzende Vorsprung 27 wirkt im Zuge des Aufsetzens der Verschlusskappe 18 auf den Mündungsstutzen 10 auf den Eingriffszahn 66 des Sperrgliedes 62 ein derart, dass hierdurch eine Schwenkverlagerung des Sperrgliedes 62 in eine Außereingriffstellung zum Zahnkranz 58 erreicht wird.

Im Zuge dieser Schwenkverlagerung gleitet der Abtastfinger 69 infolge der elastischen Ausbildung des Federarmes 68 über die, die Sperrung in dem zuvor durchgeführten Vorgang herbeiführende Kammer 32 ab und taucht hiernach in den in Bewegungsrichtung r hinter dieser Kammer belassenen Zwischenraum zwischen dieser Kammer 32 und der in Bewegungsrichtung r nachfolgenden Kammer 32. Das Sperrglied 62 ist entsprechend wieder in eine Bereitschaftsstellung zum Abtasten der nächsten Kammer 32 bei einem nächsten Verlagerungsvorgang verbracht.

Die Drehblockade der Drehhandhabe 53, die zunächst zufolge Verlagerung des Sperrgliedes 62 aufgehoben ist, ist in dieser Mündungsstutzen-Verschlussstellung übernommen durch den weiteren Vorsprung 28, der die Durchtrittsöffnung 16 durchsetzend sperrend in den Zahnring 59 der Drehhandhabe 53 eingreift.

In bevorzugter, wie auch dargestellter Ausgestaltung ist die das Sperrglied 62 in die Sperrstellung der Drehhandhabe 53 sperrende Kammer 32 nach Rückstellen des Systems zufolge Aufsetzen der Verschlusskappe 18 diejenige Kammer, deren Medikament M bei einer nächsten Drehhandhaben-Betätigung zur Freigabe angeboten wird.

Im Zuge der einzelnen Ausgaben von Medikamentenportionen beziehungsweise im Zuge der einzelnen nacheinander folgenden Inhalationen wird der Verschlussstreifen 31 sukzessive auf den Dorn 42 aufgewickelt, während der im wesentlichen mit geleerten Kammern 32 versehene Basisstreifen 30 sukzessive in die Einschubkammer 43 eingeschoben wird, in welcher der Basisstreifen 30 sich nach und nach im Wesentlichen stapelförmig ablegt.

Der Verlagerungsweg des Bandstreifens 29 je Drehhandhaben-Betätigung ist zufolge der vorgeschlagenen Lösung allein abhängig vom Abstand der Kammern 32 zueinander und weiter von diesem Abstand abgeleitet. Der Drehwinkel der Drehhandhabe 53 verringert sich im Laufe der Zunahme des Wickeldurchmessers am Dorn 42, so bevorzugt ausgehend von 90° bis zu einem verringerten Drehwinkel von etwa 70° bis 85°, weiter bevorzugt etwa 80°.

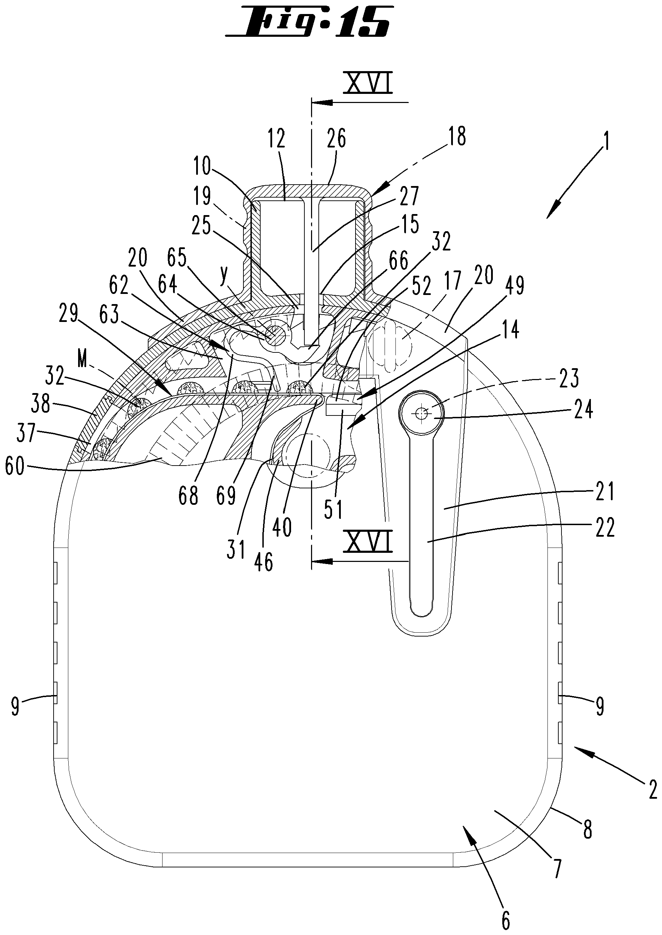

Die in Figur 15 dargestellte Stellung entspricht bevorzugt dem Auslieferungszustand der Vorrichtung 1, d.h. weiter bevorzugt dem Zustand vor einer Erstbenutzung durch den Benutzer.

Der die Kammern 32 aufweisende Bandstreifen 29 wird bevorzugt herstellerseitig in die Vorrichtung 1 eingelegt und durch Drehen der Drehhandhabe 53 so lange in Bewegungsrichtung r transportiert, bis die in Bewegungsrichtung r erste Kammer 32 das Sperrglied 62 in die, die Drehhandhabe 53 sperrende Stellung verlagert. Mit abschließendem Aufsetzen der Mündungs-Verschlusskappe 18 wird das Sperrglied 62 in die in Figur 15 dargestellte Stellung schwenkverlagert, in welcher Stellung der Abtastfinger 69 in einen Bereich zwischen der in Bewegungsrichtung betrachteten ersten Kammer und zweiten Kammer 32 einfällt. Die Drehhandhabe 53 ist entsprechend für eine Erstbenutzung durch den Benutzer nach Abnehmen der Kappe bereit. Zur Erstbenutzung ist die Handhabung der Vorrichtung 1 gleich einer jeden weiteren Benutzung.

In der Erstbenutzungsstellung gemäß Figur 15 liegt die in Bewegungsrichtung betrachtete erste Kammer 32 mit Abstand vor der Umlenkkante 40, weiter insbesondere mit Abstand zu der Durchfallöffnung 39. Entsprechend ist der dichtende Verschluss der ersten Kammer 32 durch den Verschlussstreifen 31 gegeben, wie darüber hinaus auch in jeder weiteren vorbereitenden Stellung nach Aufsetzen der Kappe auf das Mundstück bei jeder weiteren, zur nächsten Inhalation freizugebenden Kammer 32.

Der in Bewegungsrichtung r betrachtete Abstand zwischen zwei aufeinanderfolgenden Kammern 32 ist so gewählt, dass mit Verlagerung des Sperrgliedes in die Sperrstellung 62 durch eine nachfolgende Kammer 32 die der das Sperrglied betätigenden Kammer in Bewegungsrichtung r vorgelagerte Kammer 32 vom Verschlussstreifen 31 befreit über der klappenverschlossenen Durchfallöffnung 39 positioniert ist.

**Bezugszeichenliste:**

| | | | |
|---|---|---|---|
| 1 | Vorrichtung | 27 | Vorsprung |
| 2 | Gehäuse | 28 | Vorsprung |
| 3 | Gehäuseteil | 29 | Bandstreifen |
| 4 | Gehäuseboden | 30 | Basisstreifen |
| 5 | Gehäusewandung | 31 | Verschlussstreifen |
| 6 | Gehäusedeckel | 32 | Kammer |
| 7 | Gehäusedecke | 33 | Wickelkammer |
| 8 | Deckelwandung | 34 | Wickelkammerwandung |
| 9 | Greifzone | 35 | Schlitzführung |
| 10 | Mündungsstutzen | 36 | Einführabschnitt |
| 11 | Kamin | 37 | Ausbruch |
| 12 | Mündungsöffnung | 38 | Transparentbereich |
| 13 | Strömungskanal | 39 | Durchfallöffnung |
| 14 | Ausgabebereich | 40 | Umlenkkante |
| 15 | Durchtrittsöffnung | 41 | Verschlussstreifen-Wickelraum |
| 16 | Durchtrittsöffnung | 42 | Dorn |
| 17 | Einströmöffnung | 43 | Einschubkammer |
| 18 | Mündungs-Verschlusskappe | 44 | Führungsabschnitt |
| 19 | Stülpabschnitt | 45 | Wandung |
| 20 | Abschnitt | 46 | Abschnitt |
| 21 | Führungsabschnitt | 47 | Einströmkammer |
| 22 | Ausnehmung | 48 | Strömungskanalabschnitt |
| 23 | Zapfen | 49 | Klappe |
| 24 | Radialkragen | 50 | Arm |
| 25 | Durchgreifschlitz | 51 | Dichtabschnitt |
| 26 | Kappendecke | 52 | Eingriffsfortsatz |
| 53 | Drehhandhabe | M | Medikament |
| 54 | Öffnung | | |
| 55 | Stirnöffnung | a | Pfeil |
| 56 | Zapfen | b | Breite |
| 57 | Greifmulde | h | Höhe |
| | | c | Pfeil |
| 58 | Zahnkranz | r | Bewegungsrichtung |
| 59 | Zahnring | t | Tiefe |
| 60 | Zahnring | x | Drehachse |
| 61 | Sperrzahn | y | Schwenkachse |
| 62 | Sperrglied | | |
| 63 | Sperrglied-Kammer | | |
| 64 | Nabe | | |
| 65 | Achskörper | | |
| 66 | Eingriffszahn | | |
| 67 | Durchbruch | | |
| 68 | Federarm | | |
| 69 | Abtastfinger | | |
| 70 | freies Ende | | |
| 71 | Kammer | | |

## Patentansprüche

1. Vorrichtung (1) zur portionierten Ausgabe von Medikamenten (M), mit einem Bandstreifen (29), der einzelne hintereinander angeordnete Kammern (32) aufweist und in einem Gehäuse (2) aufgenommen ist, wobei der Bandstreifen (29) aus einem Basisstreifen (30) und einem Verschlussstreifen (31) besteht und erhabene Kammern (32) des Bandstreifens durch Betätigung einer Drehhandhabe (53) öffenbar sind durch scharfkantige Umlenkung des Verschlussstreifens (31), **dadurch gekennzeichnet, dass** die Betätigung der Drehhandhabe (53) über ein Sperrglied (62) durch eine in Richtung auf eine Mundstücköffnung (12) gerichtete Kammererhebung blockierbar ist, in welcher Stellung eine der blockierenden Kammererhebung bewegungsmäßig vorgeordnete, vom Verschlussstreifen (31) befreite Kammer (32) oberhalb einer Durchfallöffnung (39) liegt, welche Öffnung (39) mittels einer von einem Saugluftstrom in die Öffnungsstellung bewegbaren Klappe (49) verschließbar ist.

2. Vorrichtung nach Anspruch 1, **gekennzeichnet durch** eine Anfangs-Stopp-Stellung des Verschlussstreifens (31) kurz vor einem Erreichen der Umlenkkante (40) durch eine vorderste Kammer (32) und vor einer Betätigung des Sperrgliedes (62) durch die nachfolgende Kammer (32).

3. Vorrichtung nach einem der vorhergehenden Ansprüche, **dadurch gekennzeichnet, dass** das Sperrglied (62) zweiarmig ist.

4. Vorrichtung nach einem der vorhergehenden Ansprüche, **dadurch gekennzeichnet, dass** der Bandstreifen (29) mit den verschlossenen Kammern (32) in einer Wickelkammer (33) mit den Kammererhebungen (32) nach außen weisend aufgewickelt ist und der sich jenseits der Umlenkstelle fortsetzende Basisstreifen (30) sich in eine vergrößerte Einschubkammer (43) fortsetzt, während der Verschlussstreifen (31) auf einem von der Drehhandhabe (53) angetriebenen Dorn (42) aufwickelbar ist.

5. Vorrichtung nach einem der vorhergehenden Ansprüche, **dadurch gekennzeichnet, dass** das Sperrglied (62) in abtastender Sperrstellung so gut wie nahezu kein Drehmoment auf die Kammer-Erhebung (32) ausübt.

6. Vorrichtung nach einem der Ansprüche 4 oder 5, **dadurch gekennzeichnet, dass** das Sperrglied (62), die Durchfallöffnung (39) und der Dorn (42) auf einer Radialen der Drehhandhabe (53) hintereinander liegen.

7. Vorrichtung nach einem der vorhergehenden Ansprüche, **dadurch gekennzeichnet, dass** das abtastende Sperrglied (62) von einem Vorsprung (27) einer Mündungs-Verschlusskappe (18) in die Vorbereitungsstellung zum Abtasten der nächsten Kammer (32) zurückstellbar ist.

8. Vorrichtung nach Anspruch 7, **dadurch gekennzeichnet, dass** die Mündungs-Verschlusskappe (18) längsverschieblich und schwenkbar auf den Mündungsstutzen (10) aufsetzbar ist.

9. Vorrichtung nach einem der Ansprüche 7 oder 8, **dadurch gekennzeichnet, dass** ein Drehvorsprung (28) der Mündungs-Verschlusskappe (18) als Sperrteil der Drehhandhabe (53) zugeordnet ist.

10. Vorrichtung nach einem der Ansprüche 4 bis 9, **dadurch gekennzeichnet, dass** in Bewegungsrichtung des Basisstreifens (30) vorgelagert der Einschubkammer (43) ein die Einschieberichtung des Basisstreifens (30) in die Einschubkammer (43) vorgebender Einführabschnitt (44) ausgebildet ist.

11. Vorrichtung nach einem der vorhergehenden Ansprüche, **dadurch gekennzeichnet, dass** die Drehhandhabe (53) als großflächige Scheibe teilabdeckend vor der einen Seitenwand des Gehäuses (2) angeordnet ist.

12. Vorrichtung nach einem der vorhergehenden Ansprüche, **dadurch gekennzeichnet, dass** eine rückseitige Verzahnung (58, 59) der Drehhandhabe (53) innenseitig einen Zahnkranz (58) zum Eingriff eines Eingriffszahnes (66) aufweist, derart, dass die Haltekraft bei gestoppter Drehhandhabe auf eine Schwenkachse (y) des Sperrgliedes (62) gerichtet ist.

## Claims

1. Device (1) for the portioned output of a medication (M), with a basis strip (29) having individual chambers (32) arranged one behind the other and being received in a housing (2), consisting of a strip (30) and a closure strip (31), and the raised chambers (32) of which strip can be opened by actuating a rotary handle (53) by sharply deflecting the closure strip (31), **characterized in that** via a blocking element (62) by means of a chamber elevation directed towards the mouthpiece opening (12) the actuation of the handle (53) is blockable, in which position a chamber (32), which is arranged upstream of the blocking chamber elevation in terms of movement and being freed of the closure strip (31), is located above a fall through opening (39) which opening (39) can be closed by means of a flap (49) which can be moved into the open position by means of a suction airstream.

2. Device according to claim 1, **characterized by** an initial stop position of the closure strip (31) shortly before reaching the deflection edge (40) by a foremost chamber (32) and before an actuation of the blocking element (62) by the subsequent chamber (32).

3. Device according to either of the preceding claims, **characterized in that** the blocking element (62) has two arms.

4. Device according to any of the preceding claims, **characterized in that** the basis strip (29) with the closed chambers (32) is wound-on in a winding chamber (33), the chamber elevations (32) facing outward, and the base strip (30) continuing beyond the deflection point extends into an enlarged insertion chamber (43), while the closure strip (31) is able to be wound-on on a mandrel (42) driven by the rotary handle (53).

5. Device according to any of the preceding claims, **characterized in that**, in the sensing blocking position (Fig. 12), the blocking element 862) exerts virtually no torque on the chamber elevation (32)

6. Device according to one of the claims 4 or 5, **characterized in that** the blocking element (62), the fall-through opening (39) and the mandrel (42) are located one behind the other on a radial of the rotary handle (53).

7. Device according to any of the preceding claims, **characterized in that** the sensing blocking element (62) can be returned by a projection (27) of a mouth closure cap (18) into the preparation position, to sense the next chamber (32)

8. Device according to claim 7, **characterized in that** the mouth closure cap (18) is placeable on the mouth nozzle (10)longitudinally displaceable and suitable.

9. Device according to either claim 7 or claim 8, **characterized in that** a rotary projection (28) of the mouth closure cap (18) is related to the rotary handle (53)as a blocking part.

10. Device according to any of claims 4 to 9, **characterized in that** an introduction portion (44) predetermining the insertion direction of the base strip (30) into the introduction chamber (43) is configured upstream of the insertion chamber (43) in the movement direction of the base strip (30).

11. Device according to any of the preceding claims, **characterized in that** the rotary handle (53) is arranged in a partly overlapping manner in front of a side wall of the housing (2) as a disc having a large surface area.

12. Device according to any of the preceding claims, **characterized in that** a serration (58, 59) on the back of the rotary handle (53) has a sprocket wheel (58) on the inside for engagement with an engagement tooth (66) such that, when the rotary handle is stopped, the retention force is directed towards a pivot axis y of the blocking element (62).

## Revendications

1. Dispositif (1) pour distribuer des médicaments (M) par portions, ayant une bande (29) qui présente des chambres individuelles agencées les unes à la suite des autres (32) et qui est reçue dans un boîtier (2), dans lequel la bande (29) est constituée d'une bande de base (30) et d'une bande de fermeture (31), et des chambres surélevées (32) de la bande peuvent être ouvertes en actionnant un organe de manoeuvre rotatif (53), par déviation à angle vif de la bande de fermeture (31), **caractérisé en ce que** l'actionnement de l'organe de manoeuvre rotatif (53) peut être bloqué via un élément de blocage (62) par une saillie de chambre orientée dans la direction d'une ouverture d'embout (12), dans laquelle position une chambre (32) en amont de la saillie de chambre bloquante en terme de déplacement et libérée de la bande de fermeture (31) se trouve au-dessus d'une ouverture de passage (39), laquelle ouverture (39) peut être fermée au moyen d'un clapet (49) déplaçable dans la position ouverte par un flux d'air d'aspiration.

2. Dispositif selon la revendication 1, **caractérisé par** une position d'arrêt initiale de la bande de fermeture (31) peu avant d'atteindre le bord de déviation (40) par une chambre la plus avant (32) et avant l'actionnement de l'élément de blocage (62) par la chambre suivante (32).

3. Dispositif selon l'une des revendications précédentes, **caractérisé en ce que** l'élément de blocage (62) a deux bras.

4. Dispositif selon l'une des revendications précédentes, **caractérisé en ce que** la bande (29) est enroulée avec les chambres fermées (32) dans une chambre d'enroulement (33) avec les saillies de chambre (32) tournées vers l'extérieur, et la bande de base (30) qui continue au-delà de la position de déviation se poursuit dans une chambre d'insertion agrandie (43), tandis que la bande de fermeture (31) est enroulée sur un mandrin (42) entraîné par l'organe de manoeuvre rotatif (53).

5. Dispositif selon l'une des revendications précédentes, **caractérisé en ce que** l'élément de blocage (62), dans la position de blocage par palpage, n'exerce quasiment aucun moment de rotation sur la saillie de chambre (32).

6. Dispositif selon l'une des revendications 4 ou 5, **caractérisé en ce que** l'élément de blocage (62), l'ouverture de passage (39) et le mandrin (42) sont situés les uns derrière les autres dans une direction radiale de l'organe de manoeuvre rotatif (53) .

7. Dispositif selon l'une des revendications précédentes, **caractérisé en ce que** l'élément de blocage par palpage (62) peut être ramené en arrière dans la position de préparation pour palper la chambre suivante (32) par une saillie (27) d'un capuchon de fermeture d'embouchure (18).

8. Dispositif selon la revendication 7, **caractérisé en ce que** le capuchon de fermeture d'embouchure (18) peut être monté sur la tubulure d'embouchure (10) en étant déplaçable longitudinalement et de manière pivotante.

9. Dispositif selon l'une des revendications 7 ou 8, **caractérisé en ce qu'**une saillie de pivotement (28) du capuchon de fermeture d'embouchure (18) est associée en tant que pièce de blocage à l'organe de manoeuvre rotatif (53).

10. Dispositif selon l'une des revendications 4 à 9, **caractérisé en ce que**, en amont de la chambre d'introduction (43) dans la direction de déplacement de la bande de base (30), est agencé une partie d'admission (44) déterminant la direction d'insertion de la bande de base (30) dans la chambre d'insertion (43).

11. Dispositif selon l'une des revendications précédentes, **caractérisé en ce que** l'organe de manoeuvre rotatif (53) est agencé sous la forme d'un disque ayant une surface importante, en recouvrement partiel d'une paroi latérale du boîtier (2).

12. Dispositif selon l'une des revendications précédentes, **caractérisé en ce qu'**une denture côté arrière (58, 59) de l'organe de manoeuvre rotatif (53) comprend intérieurement une couronne dentée (58) pour venir en prise avec une dent (66) de manière que la force de maintien soit dirigée, lorsque l'organe de manoeuvre rotatif est arrêté, sur un axe de pivotement (y) de l'élément de blocage (62).
